# EUROPÄISCHE PATENTSCHRIFT

(11) **EP 2 919 654 B1**
(45) Veröffentlichungstag und Bekanntmachung des Hinweises auf die Patenterteilung: **21.02.2018**
(21) Anmeldenummer: 12794187.0
(22) Anmeldetag: 16.11.2012
(51) Int. Cl.: A61B 6/00

(54) **RÖNTGENOLOGISCHER ARBEITSPLATZ**
RADIOLOGY WORKSTATION
POSTE DE TRAVAIL RADIOLOGIQUE

(43) Veröffentlichungstag der Anmeldung: 23.09.2015
(73) Patentinhaber: Böhme Management GmbH, 06188 Landsberg OT Peißen (DE)
(72) Erfinder: BÖHME, Karl-Heinz, 06184 Kabelsketal (DE)
(74) Vertreter: Hecht, Jan-David
(86) Internationale Anmeldenummer: PCT/EP2012/004771
(87) Internationale Veröffentlichungsnummer: WO 2014/075699

(56) Entgegenhaltungen:
- DE-A1-102004 059 135
- US-A1- 2012 076 265
- US-B1- 7 810 996

## Beschreibung

Die vorliegende Erfindung betrifft einen röntgenologischen Arbeitsplatz nach dem Oberbegriff von Anspruch 1 sowie ein Verfahren zum Betrieb eines röntgenologischen Arbeitsplatzes. Röntgenologische Arbeitsplätze kommen in vielfältiger Weise zum Einsatz, vor allem zur Diagnostik und bei der Therapie in der Human- und der Tiermedizin.
Dabei kommt beispielsweise eine von einem Röntgendetektor beabstandbare Röntgenquelle zum Einsatz, um bestimmte Objekte in einem menschlichen oder tierischen Körper zu untersuchen und insbesondere dessen Lage festzustellen. Solche Arbeitsplätze weisen horizontal oder vertikal zueinander ausgerichtete Röntgenquellen und Röntgendetektoren auf.
Bekannt sind auch sogenannte C-Bögen, die im Raum verschwenkbar angeordnet sind und die einen festen Abstand zwischen Röntgenquelle und Röntgendetektor besitzen.
Außerdem ist bekannt, zwei solche C-Bögen zu verwenden, um instantan dreidimensionale Aufnahmen zu ermöglichen. Aus der US-A-2012/076265 ist ein röntgenologischer Arbeitsplatz bekannt, umfassend zumindest 2 Röntgenquellen und einen Detektor, die an einem Schienensystem angeordnet sind und die zumindest in einer Ebene verschwenkbar sind. Die Aufgabe der vorliegenden Patentanmeldung besteht darin, einen röntgenologischen Arbeitsplatz bereit zu stellen, der flexibler und/oder mit erhöhter Messgenauigkeit einsetzbar ist.
Diese Aufgabe wird gelöst mit einem röntgenologischen Arbeitsplatz nach Anspruch 1 und einem Verfahren zum Betrieb eines röntgenologischen Arbeitsplatzes nach Anspruch 15. Vorteilhafte Weiterbildungen sind in den abhängigen Unteransprüchen angegeben.
Die Erfinder haben erkannt, dass die erfindungsgemäße Aufgabe überraschend dadurch gelöst werden kann, wenn zumindest zwei Röntgenquellen und zwei Röntgendetektoren vorgesehen werden, wobei die zumindest zwei Röntgenquellen im Raum gezielt zueinander und auch gezielt in Bezug auf den zumindest einen Röntgendetektor ausrichtbar sind.

In einer bevorzugten Weiterbildung ist vorgesehen, dass zumindest eines der Elemente Röntgenquelle und Röntgendetektor frei im Raum ausrichtbar ist. Dann muss nicht die Position des Objektes an die Lage der Röntgendetektoren und Röntgenquellen angepasst werden.

Die Röntgenquellen und Röntgendetektoren sind an einem in zumindest einer Dimension, bevorzugt in zwei Dimensionen verfahrbaren Schienensystem angeordnet. Dadurch lässt sich die Position dieses Elements besonders einfach und komfortabel variieren. Dieses Schienensystem ist an einer Decke angeordnet, da dann am Boden größtmögliche Bewegungsfreiheit für das zu untersuchende Objekt und den Untersuchenden besteht.

Die Röntgenquellen und Röntgendetektoren sind in Bezug auf die Vertikale in unterschiedliche Positionen verbringbar und bevorzugt an einem in der Vertikalen teleskopierbaren Anordnungsmittel angeordnet.

Außerdem sind die Elemente Röntgenquelle und Röntgendetektor in zumindest einer Ebene verschwenkbar angeordnet, wobei der Verschwenkwinkel bevorzugt zumindest 90°, insbesondere zumindest 180° beträgt.

Es ist vorteilhaft vorsehbar, dass zumindest ein Röntgendetektor horizontal ausgerichtet, bevorzugt am Boden angeordnet ist. Dann lassen sich von stehenden und liegenden Objekten besonders einfach stereotaktische Aufnahmen hoher Qualität für beliebige Untersuchungsobjekte, insbesondere beliebiger Dimensionierung anfertigen.

Es sind zumindest zwei Röntgendetektoren vorgesehen. Damit lassen sich dreidimensionale Aufnahmen hoher Qualität für beliebige Untersuchungsobjekte, insbesondere beliebiger Dimensionierung vornehmen.

Zweckmäßig ist vorgesehen, dass zumindest eine Röntgenquelle in Bezug auf zumindest einen Röntgendetektor einen freien Abstandsbereich von zumindest 1 m bis 2,5 m, bevorzugt von zumindest 0,5 m bis 3 m und insbesondere von 0,3 m bis 5 m aufweist. Dann lassen sich neben Menschen auch kleine tierische Objekte, wie Ratten, und auch große tierische Objekte, wie Pferde und Kamele ohne weiteres mit einem einzigen röntgenologischen Arbeitsplatz untersuchen.

Besonders bevorzugt ist vorgesehen, dass zumindest eine Röntgenquelle einen Kollimator mit einer Kollimatorblende aufweist, die eine in Abhängigkeit von der Entfernung zu einer dieser Röntgenquelle zugeordneten Röntgendetektor veränderliche Öffnung aufweist, wobei die Kollimatorblende insbesondere so ansteuerbar ist, dass sie sich mit zunehmender Entfernung schließt. Dann kann die Strahlenbelastung für das Objekt minimal gehalten werden. Dabei ist zweckmäßig vorgesehen, dass die Blendenöffnung so eingestellt wird, dass eine optimale Ausleuchtung des Röntgendetektors erfolgt.

Die Sicherheit und Effizienz des röntgenologischen Arbeitsplatzes kann dadurch erhöht werden, dass eine Visiereinrichtung vorgesehen ist zur Ausrichtung zumindest einer Röntgenquelle in Bezug auf einen dieser Röntgenquelle zugeordneten Röntgendetektor, wobei der Arbeitsplatz bevorzugt so ausgebildet ist, dass der Betrieb dieser Röntgenquelle davon abhängig ist, ob diese Röntgenquelle mit diesem Röntgendetektor fluchtet. "Fluchten" meint in diesem Zusammenhang jedes gezielte Einstrahlen von Röntgenstrahlen aus einer Röntgenquelle auf einen Röntgendetektor, d.h. sowohl eine Ausrichtung des Zentralstrahls der Röntgenquelle auf das Zentrum des Röntgendetektors, als auch beispielsweise eine Ausrichtung des Zentralstrahls auf den Rand des Röntgendetektors.

Die Sicherheit und Effizienz des röntgenologischen Arbeitsplatzes kann auch dadurch erhöht werden, dass eine Beschleunigungsmessungs-, eine Bodenkraftmessungs- und/oder eine Videoanalysevorrichtung vorgesehen ist und der Arbeitsplatz so ausgebildet ist, dass der Betrieb zumindest einer Röntgenquelle davon abhängig ist, ob ein vorbestimmtes Bewegungsprofil mittels der Beschleunigungsmessung, der Bodenkraftmessung oder der Videoanalyse erkannt wird. Dadurch ist es beispielsweise möglich, diese Röntgenquelle dann zu starten, wenn ein bestimmter Bewegungsablauf beginnt, der für eine vorgesehene Bewegungsanalyse unerlässlich ist, und dann zu beenden, wenn dieser Bewegungsablauf beendet ist, so dass die Strahlenbelastung so gering wie möglich gehalten wird.

Wenn am Boden zumindest ein Laufband angeordnet ist, lassen sich besonders einfach Bewegungsanalysen vornehmen.

Insbesondere in diesem Zusammenhang ist es vorteilhaft wenn eine Vorrichtung zum Befestigen eines zu untersuchenden Objekts vorgesehen ist. Dabei kann es sich sowohl um eine fixierende Befestigung handeln, wie sie für stereotaktische Verfahren verwendet wird, um Verwacklungsfehler zu verhindern, als auch um eine mehr oder weniger lose Befestigung an Seilen, Zügeln und dgl., um Bewegungsanalysen zu ermöglichen.

Besonders vorteilhaft für Bewegungsanalysen ist es, wenn zumindest ein Röntgendetektor ein Röntgenbildverstärker ist, dem eine Hochgeschwindigkeitskamera zugeordnet ist, wobei die Hochgeschwindigkeitskamera bevorzugt ausgelegt ist größer gleich 100 Bilder/s, höchst bevorzugt größer gleich 150 Bilder/s und insbesondere größer gleich 200 Bilder/s aufzunehmen. Unabhängiger Schutz wird beansprucht für das erfindungsgemäße Verfahren zum Betrieb eines röntgenologischen Arbeitsplatzes, das sich dadurch auszeichnet, dass zumindest zwei Röntgenquellen und zwei Röntgendetektoren vorgesehen werden und die zwei Röntgenquellen im Raum gezielt zueinander und auch gezielt in Bezug auf die Röntgendetektoren ausgerichtet werden, um ein zwischen den zumindest zwei Röntgenquellen und dem zumindest einen Röntgendetektor angeordnetes Objekt zu durchleuchten. Besonders bevorzugt wird in diesem Verfahren der erfindungsgemäße röntgenologische Arbeitsplatz verwendet.

In einer bevorzugten Weiterbildung des Verfahrens ist vorgesehen, dass für stereotaktische Aufnahmen die Zentralstrahlen von zwei Röntgenquellen auf gegenüberliegende Bildfeldbegrenzungen eines zugeordneten Röntgendetektors ausgerichtet werden, wobei die beiden Röntgenquellen den selben Abstand zum Röntgendetektor aufweisen und sich zwischen den Röntgenquellen und dem Röntgendetektor ein gleichseitiges Dreieck aufspannt, dessen Basis die beiden Röntgenquellen miteinander verbindet. Das stereotaktisch zu untersuchende Objekt wird dann in dem Schnittpunkt der Zentralstrahlen angeordnet. Bei diesem Objekt handelt es sich entweder um ein gesamtes Objekt oder um den interessierenden Teil des Objektes, wie beispielsweise ein Kniegelenk eines Menschen. Insbesondere ist vorgesehen, dass die Zentralstrahlen in Bezug zur Normale des Röntgendetektors einen Winkel von 15° bis 45°, bevorzugt von 30° aufweisen.

Die Merkmale der vorliegenden Erfindung und weitere Vorteile werden im Rahmen der Beschreibung bevorzugter Ausführungsbeispiele im Zusammenhang mit den Figuren deutlich werden. Dabei zeigen rein schematisch:
- Fig. 1: den erfindungsgemäßen röntgenologischen Arbeitsplatz in einer Draufsicht von oben,
- Fig. 2: den erfindungsgemäßen röntgenologischen Arbeitsplatz nach Fig. 1 in einer Seitenansicht,
- Fig. 3: den erfindungsgemäßen röntgenologischen Arbeitsplatz nach Fig. 1 und Fig. 2 in einer ersten Verwendung,
- Fig. 4: den erfindungsgemäßen röntgenologischen Arbeitsplatz nach Fig. 1 und Fig. 2 in einer zweiten Verwendung und
- Fig. 5: den erfindungsgemäßen röntgenologischen Arbeitsplatz nach Fig. 1 und Fig. 2 in einer dritten Verwendung.

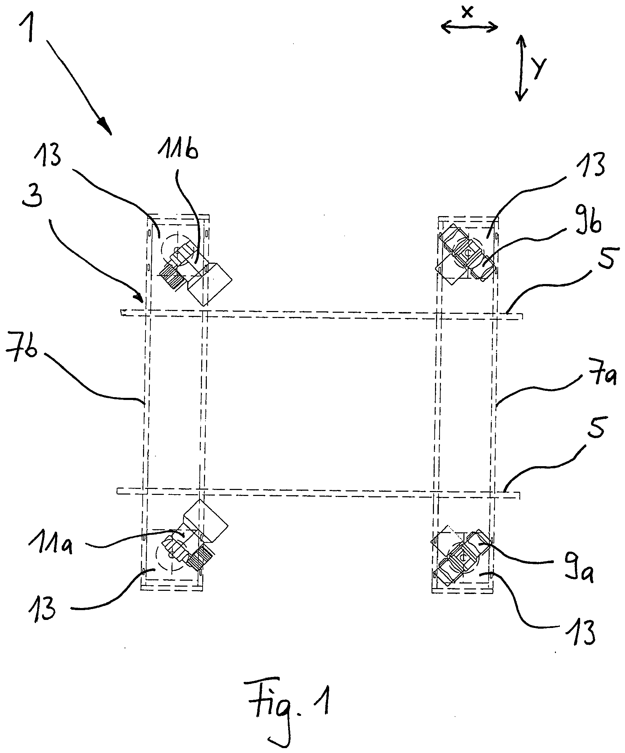

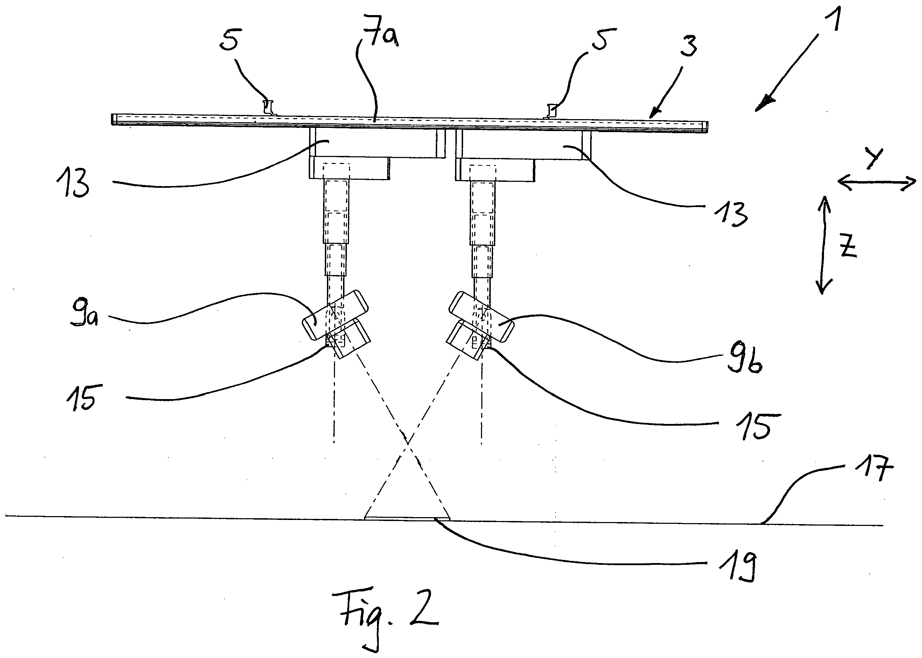

In Fig. 1 und Fig. 2 ist der erfindungsgemäße röntgenologische Arbeitsplatz 1 in einer Draufsicht von oben und in einer Seitenansicht rein schematisch gezeigt.

Es ist zu erkennen, dass der röntgenologische Arbeitsplatz 1 ein strichliert gezeichnetes Schienensystem 3 mit zwei in einer ersten Richtung x verlaufenden Schienen 5 und zwei an diesen Schienen 5 in Richtung x verfahrbar angeordneten Trägerschienen 7a, 7b aufweist, wobei an der einen Trägerschiene 7a zwei Röntgenquellen 9a, 9b und an der anderen Trägerschiene 7b zwei Röntgendetektoren 11a, 11b, wobei die Röntgenquellen 9a, 9b und auch die Röntgendetektoren 11a, 11b an den Trägerschienen 7a, 7b jeweils in einer zur Richtung x senkrechten Richtung y verfahrbar angeordnet sind. Das Schienensystem 3 ist an einer Decke befestigt und die Richtungen x, y sind dabei in einer horizontalen Ebene ausgerichtet.

Die Länge der Schienen 5 beträgt beispielsweise ca. 5 m und die Trägerschienen 7a, 7b weisen eine Länge von ca. 4 m und eine Breite von ca. 55 cm auf, wobei der Abstand der Schienen 5 voneinander ca. 1,9 m beträgt. Diese Elemente sind vorteilhaft aus Edelstahl gefertigt.

Die Anordnung der Röntgenquellen 9a, 9b und der Röntgendetektoren 11a, 11b an den jeweiligen Trägerschienen 7a, 7b erfolgt dabei, wie in Fig. 2 besonders gut zu erkennen ist, über jeweils einen in einer senkrecht zu den Richtungen x, y orientierten Richtung z teleskopierbaren Vertikalträger 13, an dem die jeweilige Röntgenquelle 9a, 9b bzw. Röntgendetektor 11a, 11b um eine vertikale Achse 360° verdrehbar und in einer vertikalen Ebene um 360° verschwenkbar angeordnet ist. Hierzu sind jeweilige Verdreh-Verkippungsmittel 15 vorgesehen.

Die Röntgenquellen 9a, 9b sind Röntgenquellen bekannter Bauart, wie sie beispielsweise aus C-Bögen bekannt sind. Die Röntgendetektoren 11a, 11b beinhalten jeweils einen Röntgenbildverstärker (nicht gezeigt) bekannten Aufbaus und eine Hochgeschwindigkeitsvideokamera (nicht gezeigt), die in der Lage ist, bis zu 250 Bilder/s aufzunehmen.
Wie in Fig. 2 zu erkennen ist, ist am Boden 17 an weiterer Röntgendetektor 19 angeordnet, dessen Detektorfläche (nicht gezeigt) sich horizontal erstreckt. Dieser Röntgendetektor 19 kann als Röntgenfolie ausgebildet sein, ist jedoch bevorzugt ebenfalls ein elektronischer Detektor.
Die Röntgenquellen 9a, 9b weisen jeweils einen Kollimator (nicht gezeigt) auf, dessen Kollimatorblende (nicht gezeigt) in ihrer Öffnung exakt und vollautomatisch so an die exakte Entfernung von dem jeweiligen Röntgendetektor 11a, 11b, 19 anpassbar ist, dass für eine fluchtende Anordnung das ausgestrahlte Strahlenbündel der Röntgenquelle 9a, 9b den Bildbereich des Röntgendetektors 11a, 11b, 19 abdeckt. Für eine stereotaktische Aufnahme ist vorgesehen, dass der Zentralstrahl einer Röntgenquelle 9a, 9b auf den gegenüberliegenden Bildrand des jeweiligen Röntgendetektors 11a, 11b, 19 eingestellt wird und der Kollimator so eingestellt ist, dass das ausgestrahlte Strahlenbündel bis zum anderen Bildrand reicht. Zur Verringerung der Strahlenbelastung kann zusätzlich vorgesehen sein, dass die Kollimatorblende in zumindest einer Richtung so asymmetrisch verfahrbar ist, dass der neben dem Zentralstrahl befindlich Teil des Strahlenbündels, der nicht auf den Röntgendetektor 11a, 11b, 19 fallen würde, ausgeblendet wird. Vorteilhaft ist die Kollimatorblende in beiden Richtungen asymmetrisch einstellbar. Für die vollautomatische Betätigung der Kollimatorblende wird die genaue Lage der Elemente zueinander im Raum mittels besonderer Positionssensoren (nicht gezeigt) bestimmt.

Weiterhin sind folgende, nicht näher gezeigte Elemente an dem erfindungsgemäßen röntgenologischen Arbeitsplatz 1 vorhanden: ein Laufband sowie eine Befestigungsvorrichtung, die beide am Boden 17 angeordnet sind. Außerdem sind Anzeigegeräte zur Auswertung vorhanden und es ist eine Steuer- und Auswertevorrichtung vorgesehen, um die vorzunehmenden Messungen durchzuführen und anschließend oder währenddessen eine Analyse vornehmen zu können. An jeder Röntgenquelle 9a, 9b ist ein optisches Visiermittel in Form eines Laserstrahlers angeordnet und an den Röntgendetektoren 11a, 11b jeweils ein oder mehrere Lichtdetektoren, um festzustellen, ob eine Röntgenquelle 9a, 9b auf den gewünschten Röntgendetektor 11, 19 optimal ausgerichtet ist. Dabei kann es sich um eine fluchtende Ausrichtung handeln, wie sie für dreidimensionale Aufnahmen erforderlich ist, oder um eine auf ein Objekt fokussierte Ausrichtung, wie sie für stereotaktische Aufnahmen erforderlich ist.

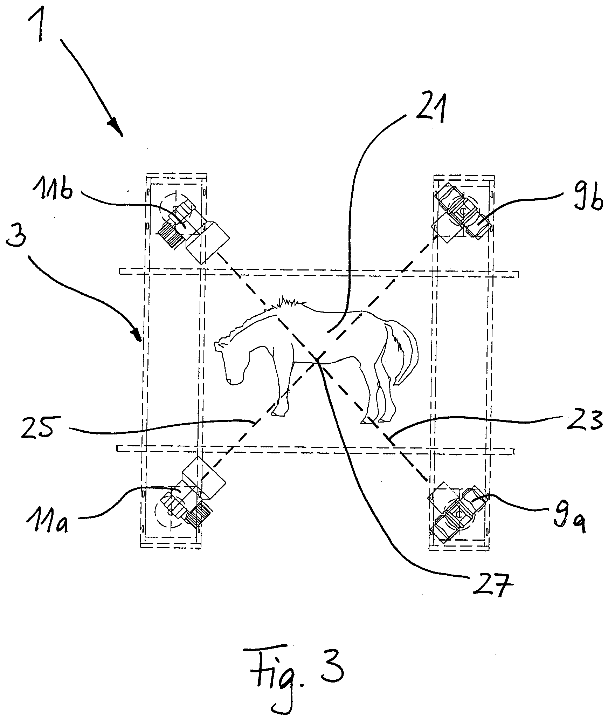

In Fig. 3 ist ein erster bevorzugter Anwendungsfall näher gezeigt. Dabei steht ein Pferd 21 in dem erfindungsgemäßen röntgenologischen Arbeitsplatz 1 auf dem Laufband und ist so befestigt, dass es sich zwar bewegen, beispielsweise traben, jedoch das Laufband nicht verlassen kann.

Die linke Röntgenquelle 9a ist mit Hilfe der Visiervorrichtung fluchtend auf den rechten Röntgendetektor 11b ausgerichtet und die rechte Röntgenquelle 9a ist mit Hilfe der Visiervorrichtung fluchtend auf den linken Röntgendetektor 11b ausgerichtet (d.h. die Zentralstrahlen der beiden Röntgenquellen 9a, 9b sind auf das Zentrum der jeweiligen Röntgendetektoren 11a, 11b ausgerichtet). Die Visiervorrichtung ist dabei so mit der Steuer- und Auswerteeinheit verbunden, dass ein Betrieb der Röntgenquellen 9a, 9b nur dann möglich ist, wenn die Fluchtung vorliegt, wobei in diesem Fall die beiden Fluchtungsstrahlen 23, 25 jeweils zentral auf die beiden Röntgendetektoren 11b, 11a eintreffen. Die beiden Fluchtungsstrahlen 23, 25 kreuzen sich in einem Schnittpunkt 27, in dem das Pferd 21 auf dem Laufband steht, d.h. die beiden Fluchtungsstrahlen 23, 25 wurden in den Richtungen x, y, z so ausgereichtet, dass der Schnittpunkt 27 auf das interessierende Teil des Pferdes, beispielsweise ein Gelenk ausgerichtet ist. (Die genaue Ausrichtung des Schnittpunktes 27 auf das Pferd 21 ist hier nicht exakt gezeigt.)

Das Laufband ist mit Bodenkraftsensoren ausgerüstet, um ein bestimmtes Bewegungsmuster des Pferdes zu erkennen und auch einen Startpunkt und eine Endpunkt dieses Bewegungsmusters zu erkennen. Bei dem Bewegungsmuster kann es sich beispielsweise um ein Traben oder dgl. handeln. Alternativ oder zusätzlich können für dieses Erkennen Beschleunigungssensoren und/oder eine Videokammara verwendet werden.

Sobald das Bewegungsmuster und der Startpunkt erkannt wurden, wird die röntgenologische Durchleuchtungsaufnahme gestartet und kontinuierlich durchgeführt und nach Erkennen des Endpunktes wird die Durchleuchtung beendet. Üblicherweise wird die Durchleuchtung für zumindest zwei, bevorzugt zumindest drei und insbesondere fünf Wiederholungen des Bewegungsmusters durchgeführt. Dadurch wird eine definierte Durchleuchtung sichergestellt und gleichzeitig die Strahlenbelastung zu minimieren.

Durch die Analyse der Bewegung können beispielsweise Fehlstände von Gelenken erfasst werden. Bei Menschen können so Knie-, Hüft- und Fußgelenke untersucht werden. Bei Rindern und Schafen kann beispielsweise der Klauenschnitt so optimiert werden, dass die Bewegung optimal verläuft.

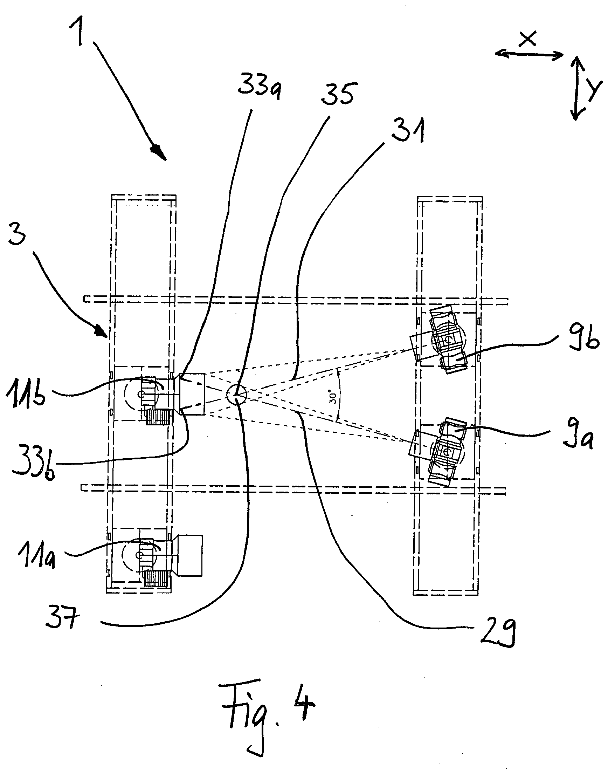

In Fig. 4 ist ein zweiter bevorzugter Anwendungsfall näher gezeigt. Dabei sind die beiden Röntgenquellen 9a, 9b mit ihren Zentralstrahlen 29, 31 auf die jeweils gegenüberliegenden Bildfeldgrenzen 33a, 33b des Röntgendetektors 11b ausgerichtet, der lotrecht in Bezug auf die Verbindungslinie der beiden Röntgenquellen 9a, 9b angeordnet wird, wobei sich ein gleichseitiges Dreieck zwischen den drei Elementen Röntgenquellen 9a, 9b und Röntgendetektor 11b aufspannt und die Abstände der Röntgenquellen 9a, 9b vom Röntgendetektor 11b identisch sind. In den Schnittpunkt 35 der Zentralstrahlen 29, 31 wird das zu untersuchende Objekt 37 verbracht, so dass beide Röntgenquellen 9a, 9b auf dieses Objekt 37 fokussiert sind. Die Zentralstrahlen der beiden Röntgenquellen 9a, 9b scheiden sich unter einem Winkel von 30°, d.h. sie sind in Bezug auf die Normale des Röntgendetektors 11b mit einem Winkel von jeweils 15° angeordnet. Zur Befestigung des Objektes 37 ist eine reproduzierende Fixierung vorgesehen, so dass stereotaktische Durchleuchtung hoher Qualität vorgenommen werden können. In diesem Fall finden die Durchleuchtungen in einer horizontal verlaufenden Ebene statt. Diese Variante eignet sich vor allem für stehende Objekte.

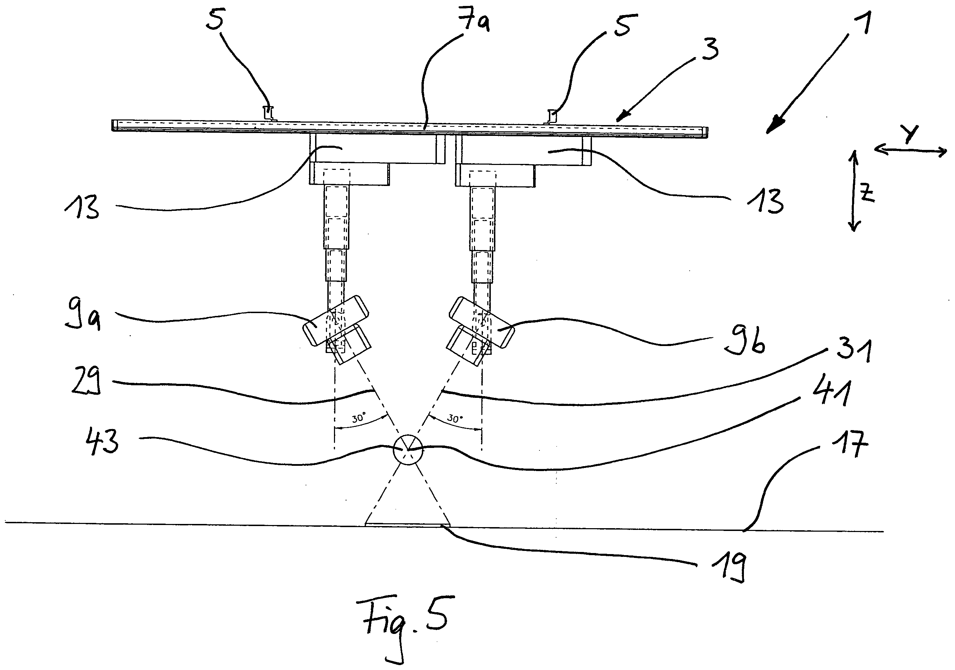

In Fig. 5 ist ein dritter bevorzugter Anwendungsfall näher gezeigt. Dabei sind die beiden Röntgenquellen 9a, 9b mit ihren Zentralstrahlen 29, 31 auf die jeweils gegenüberliegenden Bildfeldgrenzen 39a, 39b des Röntgendetektors 19 ausgerichtet, wobei die beiden Röntgenquellen 9a, 9b so verschwenkt sind, dass sie zusammen mit dem Röntgendetektor 19 eine vertikal ausgerichtete Ebene aufspannen und sich ein gleichseitiges Dreieck zwischen den drei Elementen Röntgenquellen 9a, 9b und Röntgendetektor 19 aufspannt und die Abstände der Röntgenquellen 9a, 9b vom Röntgendetektor 19 identisch sind. In den Schnittpunkt 41 der Zentralstrahlen 29, 31 wird durch genaue Justierung in den Richtungen x, y, z das zu untersuchende Objekt 43 verbracht, so dass beide Röntgenquellen 9a, 9b auf dieses Objekt 43 fokussiert sind. Die Zentralstrahlen der beiden Röntgenquellen 9a, 9b sind in Bezug auf die Normale des Röntgendetektors 19 mit einem Winkel von 30° angeordnet. Zur Befestigung des Objektes 43 ist wiederum eine reproduzierende Fixierung vorgesehen, so dass stereotaktische Durchleuchtungen hoher Qualität vorgenommen werden können. Diese Variante eignet sich vor allem für liegende Objekte, wobei ggf. eine geeignete Hubeinrichtung, die für die Röntgenstrahlung transparent ausgebildet ist, vorgesehen werden muss, um das Objekt in den Schnittpunkt 41 verbringen zu können.

Aus der vorstehenden Darstellung ist deutlich geworden, dass der erfindungsgemäße röntgenologische Arbeitsplatz sehr flexibel einsetzbar ist und Durchleuchtungen hoher Qualität zu liefern im Stande ist.

## Patentansprüche

1. Röntgenologischer Arbeitsplatz (1) mit zumindest einer Röntgenquelle (9a, 9b) und zumindest einem Röntgendetektor (11a, 11b, 19), wobei zumindest zwei Röntgenquellen (9a, 9b) und zumindest zwei Röntgendetektoren (11a, 11b, 19) vorgesehen sind, **dadurch gekennzeichnet, dass** zwei Röntgenquellen (9a, 9b) und zwei Röntgendetektoren (11a, 11b) an einem an einer Decke angeordneten Schienensystem (3) in zwei Dimensionen (x, y) einer horizontalen Ebene verfahrbar angeordnet sind, wobei die zwei Röntgenquellen (9a, 9b) im Raum gezielt zueinander und auch gezielt in Bezug auf die zwei Röntgendetektoren (11a, 11b) ausrichtbar sind, wobei die zwei Röntgenquellen (9a, 9b) und die zwei Röntgendetektoren (11a, 11b) jeweils um eine vertikale Achse verdrehbar, jeweils in einer vertikalen Ebene verschwenkbar und jeweils in Bezug auf die Vertikale (z) in unterschiedliche Positionen verbringbar angeordnet sind, so dass eine erste Röntgenquelle (9a) der zwei Röntgenquellen (9a, 9b) fluchtend auf einen Röntgendetektor (11b) der zwei Röntgendetektoren (11a, 11b) und eine zweite Röntgenquelle (9b) der zwei Röntgenquellen (9a, 9b) fluchtend auf einen Röntgendetektor (11a) der zwei Röntgendetektoren (11a, 11b) unter Kreuzung der entsprechenden Fluchtungsstrahlen (23, 25) in einem Schnittpunkt (27) ausrichtbar sind, um ein zwischen den zwei Röntgenquellen (9a, 9b) und den zwei Röntgendetektoren (11a, 11b, 19) angeordnetes Objekt (21, 37, 43) im Schnittpunkt (27) zu durchleuchten.

2. Röntgenologischer Arbeitsplatz (1) nach Anspruch 1, **dadurch gekennzeichnet, dass** der Verschwenkungswinkel zumindest 90°, bevorzugt zumindest 180°, insbesondere 360° beträgt.

3. Röntgenologischer Arbeitsplatz (1) nach einem der vorherigen Ansprüche, **dadurch gekennzeichnet, dass** zumindest eines der Elemente Röntgenquelle (9a, 9b) und Röntgendetektor (11a, 11b) an einem in der Vertikalen (z) teleskopierbaren Anordnungsmittel (13) angeordnet ist.

4. Röntgenologischer Arbeitsplatz (1) nach einem der vorherigen Ansprüche, **dadurch gekennzeichnet, dass** ein weiterer Röntgendetektor (19) horizontal ausgerichtet, bevorzugt am Boden (17) angeordnet ist.

5. Röntgenologischer Arbeitsplatz (1) nach einem der vorherigen Ansprüche, **dadurch gekennzeichnet, dass** zumindest eine Röntgenquelle (9a, 9b) in Bezug auf zumindest einen Röntgendetektor (11a, 11b, 19) einen freien Abstandsbereich von zumindest 1 m bis 2,5 m, bevorzugt von zumindest 0,5 m bis 3 m und insbesondere von 0,3 m bis 3 m aufweist.

6. Röntgenologischer Arbeitsplatz (1) nach einem der vorherigen Ansprüche, **dadurch gekennzeichnet, dass** zumindest eine Röntgenquelle (9a, 9b) einen Kollimator mit einer Kollimatorblende aufweist, die eine in Abhängigkeit von der Entfernung zu einer dieser Röntgenquelle (9a, 9b) zugeordneten Röntgendetektor (11a, 11b, 19) veränderliche Öffnung aufweist, wobei die Kollimatorblende bevorzugt so ansteuerbar ist, dass sie sich mit zunehmender Entfernung schließt.

7. Röntgenologischer Arbeitsplatz (1) nach einem der vorherigen Ansprüche, **dadurch gekennzeichnet, dass** eine Visiereinrichtung vorgesehen ist zur Ausrichtung zumindest einer Röntgenquelle (9a, 9b) in Bezug auf einen dieser Röntgenquelle (9a, 9b) zugeordneten Röntgendetektor (11a, 11b, 19), wobei der Arbeitsplatz (1) bevorzugt so ausgebildet ist, dass der Betrieb dieser Röntgenquelle (9a, 9b) davon abhängig ist, ob diese Röntgenquelle (9a, 9b) mit diesem Röntgendetektor (11a, 11b, 19) fluchtet.

8. Röntgenologischer Arbeitsplatz (1) nach einem der vorherigen Ansprüche, **dadurch gekennzeichnet, dass** eine Beschleunigungsmessungs-, eine Bodenkraftmessungs- und/oder eine Videoanalysevorrichtung vorgesehen ist und der Arbeitsplatz (1) so ausgebildet ist, dass der Betrieb zumindest einer Röntgenquelle (9a, 9b) davon abhängig ist, ob ein vorbestimmtes Bewegungsprofil mittels der Beschleunigungsmessung, der Bodenkraftmessung oder der Videoanalyse erkannt wird.

9. Röntgenologischer Arbeitsplatz (1) nach einem der vorherigen Ansprüche, **dadurch gekennzeichnet, dass** am Boden (17) zumindest ein Laufband angeordnet ist.

10. Röntgenologischer Arbeitsplatz (1) nach einem der vorherigen Ansprüche, **dadurch gekennzeichnet, dass** eine Vorrichtung zum Befestigen eines zu untersuchenden Objekts (21, 37, 43) vorgesehen ist.

11. Röntgenologischer Arbeitsplatz (1) nach einem der vorherigen Ansprüche, **dadurch gekennzeichnet, dass** zumindest ein Röntgendetektor (11a, 11b, 19) ein Röntgenbildverstärker ist, dem eine Hochgeschwindigkeitskamera zugeordnet ist, wobei die Hochgeschwindigkeitskamera bevorzugt ausgelegt ist größer gleich 100 Bilder/s, höchst bevorzugt größer gleich 150 Bilder/s und insbesondere größer gleich 200 Bilder/s aufzunehmen.

12. Verfahren zum Betrieb eines röntgenologischen Arbeitsplatzes (1), wobei zumindest zwei Röntgenquellen (9a, 9b) und zumindest zwei Röntgendetektoren (11a, 11b, 19) vorgesehen werden, **dadurch gekennzeichnet, dass** die zumindest zwei Röntgenquellen (9a, 9b) und die zumindest zwei Röntgendetektoren (11a, 11b, 19) an einem an einer Decke angeordneten Schienensystem (3) in zwei Dimensionen (x, y) einer horizontalen Ebene verfahrbar angeordnet sind, wobei die zwei Röntgenquellen (9a, 9b) im Raum gezielt zueinander und auch gezielt in Bezug auf die zumindest zwei Röntgendetektoren (11a, 11b, 19) ausgerichtet werden, wobei die Röntgenquellen (9a, 9b) und Röntgendetektoren (11a, 11b) jeweils um eine vertikale Achse verdrehbar, jeweils in einer vertikalen Ebene verschwenkbar und jeweils in Bezug auf die Vertikale (z) in unterschiedliche Positionen verbringbar angeordnet sind und eine erste Röntgenquelle (9a) der zumindest zwei Röntgenquellen (9a, 9b) fluchtend auf einen Röntgendetektor (11b) der zumindest zwei Röntgendetektoren (11a, 11b, 19) und eine zweite Röntgenquelle (9b) der zumindest zwei Röntgenquellen (9a, 9b) fluchtend auf einen Röntgendetektor (11a) der zumindest zwei Röntgendetektoren (11a, 11b, 19) unter Kreuzung der entsprechenden Fluchtungsstrahlen (23, 25) in einem Schnittpunkt (27) ausgerichtet werden, um ein zwischen den zwei Röntgenquellen (9a, 9b) und den zwei Röntgendetektoren (11a, 11b) angeordnetes Objekt (21, 37, 43) im Schnittpunkt (27) zu durchleuchten.

13. Verfahren nach Anspruch 12, **dadurch gekennzeichnet, dass** der röntgenologische Arbeitsplatz (1) mit den Merkmalen eines der Ansprüche 1 bis 12 ausgebildet ist.

## Claims

1. An X-ray work station (1), comprising at least one X-ray source (9a, b) and at least one X-ray detector (11a, 11b, 19) wherein at least two X-ray sources (9a, 9b) and at least two X-ray detectors (11a, b, 19) are provided, **characterized in that** two X-ray sources and two X-ray detectors (11a, 11b) are arranged at a rail system (3) that is arranged at a ceiling so that the two X-ray sources and the X-ray detectors are movable in two dimensions (X, Y) of a horizontal plane, wherein the two X-ray sources (9a, b) are alignable relative to one another in space in a controlled manner and are also alignable in a controlled manner relative to the two X-ray detectors (11a, b) wherein the two X-ray sources (9a, b) and the two X-ray detectors (11a, b) are respectively arranged rotatable about a vertical axis, respectively pivotable in a vertical plane and respectively movable in different positions with respect to a vertical (Z) so that a first X-ray source (9a) of the two X-ray sources (9a, b) is alignable with an X-ray detector (11b) of the two X-ray detectors (11a, b) and a second X-ray source (9b) of the two X-ray sources (9a, b) is alignable with an X-ray detector (11a) of the two X-ray detectors (11a, b) so that respective alignment beams (23, 25) intersect in an intersection point (27) so that an object (21, 37, 43) arranged between the two X-ray sources (9a, b) and two X-ray detectors (11a, b) in the intersection point (27) is X-rayed.

2. The X-ray work station (1) according to claim 1, **characterized in that** a pivot angle is at least 90º, advantageously at least 180º, in particular 360º.

3. The X-ray work station (1) according to one of the preceding claims, **characterized in that** at least one of the elements X-ray source (9a, b) and X-ray detector (11a, b) is arranged at an arrangement device (13) that is telescopable in the vertical (Z)..

4. The X-ray work station (1) according to one of the preceding claims, **characterized in that** an additional X-ray detector (19) is oriented horizontally and advantageously arranged at a floor (17).

5. The X-ray work station (1) according to one of the preceding claims, **characterized in that** at least one X-ray source (9a, b) has a clear distance of at least 1 meter to 2.5 meters advantageously at least 0.5 meters to 3 meters and in particular 0.3 meters to 3 meters with reference to at least one X-ray detector (11a, 11b, 19).

6. The X-ray work station (1) according to one of the preceding claims, **characterized in that** at least one X-ray source (9a, b) includes a collimator with a collimator aperture which includes an opening that is variable as a function of a distance from an X-ray detector (11a, 11b, 19) that is associated with the X-ray source (9a, b), wherein the collimator aperture is advantageously controllable so that it closes with an increasing distance.

7. The X-ray work station (1) according to one of the preceding claims, **characterized in that** a sight is provided to align at least one X-ray source (9a, b) with respect to an X-ray detector (11a, 11b, 19) that is associated with this X-ray source (9a, 9b), wherein the work station (1) is advantageously configured so that operations of the X-ray source (9a, b) depend on the X-ray source (9a, b) being aligned with the X-ray detector.

8. The X-ray work station (1) according to one of the preceding claims, **characterized in that** an acceleration measurement, a ground force measurement and/or a video analysis device is provided and the work station (1) is configured so that operations of at least one X-ray source (9a, b) are a function of a predetermined movement profile being detected by the acceleration measurement, the ground force measurement or the video analysis.

9. The X-ray work station (1) according to one of the preceding claims, **characterized in that** at least one running band is arranged at the floor (17).

10. The X-ray work station (1) according to one of the preceding claims, **characterized in that** a device for attaching an object (21, 37, 43) to be examined is provided.

11. The X-ray work station (1) according to one of the preceding claims, **characterized in that** at least one X-ray detector (11a, 11b, 19) is an X-ray image amplifier that is associated with a high speed camera wherein the high speed camera is advantageously configured to take at least 100 images per second, more advantageously at least 150 images per second and in particular at least 200 images per second.

12. A method for operating a X-ray work station (1), wherein at least two X-ray sources (9a, 9b) and at least two X-ray detectors (11a, b, 19) are provided, **characterized in that** two X-ray sources and two X-ray detectors (11a, 11b) are arranged at a rail system (3) that is arranged at a ceiling so that the two X-ray sources and the X-ray detectors are movable in two dimensions (X, Y) of a horizontal plane, wherein the two X-ray sources (9a, b) are alignable relative to one another in space in a controlled manner and are also alignable in a controlled manner relative to the two X-ray detectors (11a, b) wherein the two X-ray sources (9a, b) and the two X-ray detectors (11a, b) are respectively arranged rotatable about a vertical axis, respectively pivotable in a vertical plane and respectively movable in different positions with respect to a vertical (Z) so that a first X-ray source (9a) of the two X-ray sources (9a, b) is alignable with an X-ray detector (11b) of the two X-ray detectors (11a, b) and a second X-ray source (9b) of the two X-ray sources (9a, b) is alignable with an X-ray detector (11a) of the two X-ray detectors (11a, b) so that respective alignment beams (23, 25) intersect in an intersection point (27) so that an object (21, 37, 43) arranged between the two X-ray sources (9a, b) and two X-ray detectors (11a, b) in the intersection point (27) is X-rayed.

13. The method according to claim 12, **characterized in that** the X-ray work station (1) is configured with the features of one of the claims 1 through 12.

## Revendications

1. Poste de travail radiologique (1) possédant au moins une source de rayons X (9a, 9b) et au moins un détecteur de rayons X (11a, 11b, 19), dans lequel au moins deux sources de rayons X (9a, 9b) et au moins deux détecteurs de rayons X (11a, 11b, 19) sont prévus, **caractérisé en ce que** deux sources de rayons X (9a, 9b) et deux détecteurs de rayons X (11a, 11b, 19) sont arrangés pour être mobiles sur un système de rails prévu au plafond, selon deux dimensions (x, y) d'un plan horizontal, et dans lequel les deux sources de rayons X (9a, 9b) sont orientables dans l'espace l'une par rapport à l'autre et aussi par rapport aux deux détecteurs de rayons X (11a, 11b), et dans lequel les deux sources de rayons X (9a, 9b) et les deux détecteurs de rayons X (11a, 11b) sont arrangés pour pouvoir respectivement tourner autour d'un axe vertical, pivoter dans un plan vertical et se déplacer sur différentes positions en relation à l'axe vertical (z), de sorte qu'une première source de rayons X (9a) des deux sources de rayons X (9a, 9b) est orientable pour être alignée sur un détecteur de rayons X (11b) parmi deux détecteurs de rayons X (11a, 11b) et une seconde source de rayons X (9b) des deux sources de rayons X (9a, 9b) est orientable pour être alignée sur un détecteur de rayons X (11a) parmi deux détecteurs de rayons X (11a, 11b) pour que les faisceaux de rayons X correspondants (23, 25) se coupent en un point d'intersection (27), afin de cribler un objet (21, 37, 43) disposé entre les deux sources de rayons X (9a, 9b) et les deux détecteurs de rayons X (11a, 11b, 19) au point d'intersection (27).

2. Poste de travail radiologique (1) selon la revendication 1, **caractérisé en ce que** l'angle de pivot est d'au moins 90º, de préférence d'au moins 180º, en particulier de 360º.

3. Poste de travail radiologique (1) selon l'une des revendications précédentes, **caractérisé en ce qu'**au moins un des éléments sources de rayons X (9a, 9b) et détecteurs de rayons X (11a, 11b) est arrangé sur un moyen de positionnement (13) télescopique à la verticale (z).

4. Poste de travail radiologique (1) selon l'une des revendications précédentes, **caractérisé en ce qu'**un détecteur de rayons X supplémentaire (19) est orienté horizontalement, de préférence arrangé sur le sol (17).

5. Poste de travail radiologique (1) selon l'une des revendications précédentes, **caractérisé en ce qu'**au moins une source de rayons X (9a, 9b) comporte par rapport à au moins un détecteur de rayons X (11a, 11b, 19) une distance libre dans un intervalle d'au moins 1 m à 2,5 m, de préférence d'au moins 0,5 m à 3 m, et en particulier de 0,3 m à 3 m.

6. Poste de travail radiologique (1) selon l'une des revendications précédentes, **caractérisé en ce qu'**au moins une source de rayons X (9a, 9b) comporte un collimateur possédant une grille de collimation, comportant une ouverture variable en fonction de l'écart par rapport à un détecteur de rayons X (11a, 11b, 19) affecté à cette source de rayons X (9a, 9b), dans lequel la grille de collimation est de préférence commandable de sorte qu'elle se ferme lorsque l'écart augmente.

7. Poste de travail radiologique (1) selon l'une des revendications précédentes, **caractérisé en ce qu'**un dispositif de visée est prévu pour orienter au moins une source de rayons X (9a, 9b) par rapport à un détecteur de rayons X (11a, 11b, 19) affecté à cette source de rayons X (9a, 9b), dans lequel le poste de travail (1) est de préférence conçu de sorte que l'opération de cette source de rayons X (9a, 9b) dépende de l'alignement de cette source de rayons X (9a, 9b) avec ce détecteur de rayons X (11a, 11b, 19).

8. Poste de travail radiologique (1) selon l'une des revendications précédentes, **caractérisé en ce qu'**un dispositif de mesure d'accélération, un dispositif de mesure de la force exercée sur le sol et/ou un dispositif d'analyse vidéo est prévu et que le poste de travail est conçu de sorte que l'opération d'au moins une source de rayons X (9a, 9b) dépende de la reconnaissance d'un profil de mouvement prédéfini au moyen du dispositif de mesure d'accélération, du dispositif de mesure de la force exercée sur le sol ou du dispositif d'analyse vidéo.

9. Poste de travail radiologique (1) selon l'une des revendications précédentes, **caractérisé en ce qu'**au moins un tapis roulant est arrangé sur le sol (17).

10. Poste de travail radiologique (1) selon l'une des revendications précédentes, **caractérisé en ce qu'**un dispositif de fixation d'un objet à examiner (21, 37, 43) est prévu.

11. Poste de travail radiologique (1) selon l'une des revendications précédentes, **caractérisé en ce qu'**au moins un détecteur de rayons X (11a, 11b, 19) est un amplificateur de brillance radiologique, affecté à une caméra haute vitesse, dans lequel la caméra haute vitesse est conçue avec une vitesse supérieure à 100 images/s, de préférence supérieure à 150 images/s, et en particulier supérieure à 200 images/s.

12. Procédé pour l'opération d'un poste de travail radiologique (1), dans lequel au moins deux sources de rayons X (9a, 9b) et au moins deux détecteurs de rayons X (11a, 11b, 19) sont prévus, **caractérisé en ce que** les deux sources de rayons X (9a, 9b) au moins et les deux détecteurs de rayons X (11a, 11b, 19) au moins sont arrangés pour être mobiles sur un système de rails prévu au plafond, selon deux dimensions (x, y) d'un plan horizontal, et dans lequel les deux sources de rayons X (9a, 9b) sont orientées dans l'espace l'une par rapport à l'autre et aussi par rapport aux deux détecteurs de rayons X (11a, 11b, 19) au moins, et dans lequel les sources de rayons X (9a, 9b) et les détecteurs de rayons X (11a, 11b) sont arrangés pour pouvoir respectivement tourner autour d'un axe vertical, pivoter dans un plan vertical et se déplacer sur différentes positions en relation à l'axe vertical (z), et une première source de rayons X (9a) parmi deux sources de rayons X au moins (9a, 9b) est orientée pour être alignée sur un détecteur de rayons X (11b) parmi deux détecteurs de rayons X (11a, 11b, 19) au moins et une seconde source de rayons X (9b) des deux sources de rayons X (9a, 9b) au moins est orientée pour être alignée sur un détecteur de rayons X (11a) parmi deux détecteurs de rayons X (11a, 11b, 19) au moins pour que les faisceaux de rayons X correspondants (23, 25) se coupent en un point d'intersection (27), afin de cribler un objet (21, 37, 43) disposé entre les deux sources de rayons X (9a, 9b) et les deux détecteurs de rayons X (11a, 11b) au point d'intersection (27).

13. Procédé selon la revendication 12, **caractérisé en ce que** le poste de travail radiologique (1) est conçu avec les caractéristiques d'une des revendications 1 à 12.
